# EUROPEAN PATENT APPLICATION

(11) **EP 3 363 414 A1**
(43) Date of publication of application: **22.08.2018**
(21) Application number: 17168988.8
(22) Date of filing: 02.05.2017
(51) Int. Cl.: A61F 7/02, A61F 7/10

(54) **MANUFACTURING METHOD FOR ICE/HOT COMPRESS PRODUCT**

(30) Priority: 15.02.2017 US 201715433856
(71) Applicant: Taiwan Stanch Co., Ltd., Taipei City 10473 (TW)
(72) Inventor: Huang, Chun-Sheng, 10473 Taipei City (TW)
(74) Representative: Ruschke, Hans Edvard

(57) **Abstract**

A manufacturing method for an ice/hot compress product includes the following steps: providing a polyester fiber cloth as an outer layer (10); adding a thermochromic material (11) into the outer layer (10) when the outer layer (10) is dyed; and attaching an inner layer (12) to an inner side of the outer layer (10) to which the thermochromic material (11) has been added to form a composite-layer material; manufacturing a container (20) through the composite-layer material.

## Description

### BACKGROUND OF THE INVENTION

### Field of the Invention

The invention relates to a manufacturing method for an ice/hot compress product, and more particularly to a manufacturing method for an ice/hot container with thermochromic function or an n ice/hot pack with thermochromic function, which simplifies a procedure for manufacturing the ice/hot compress product.

### Description of the Related Art

Human body has a normal temperature of 37°C. However, disease or sport injuries can raise human's body temperature. For example, when a person catches a cold, leucocytes in his/her blood may release endogenous pyrogen which causes rising of his/her body temperature. The sport injuries may cause muscle inflammation or swelling so as to raise local body temperature. The raised body temperature can be reduced by ice compress which prevents the high body temperature from hurting his/her body.

In addition, muscle ache caused by long-term working or hyper kinesis is ceased by hot compress. The optimal temperature of hot compress is 40°C -50°C. When a hot towel is used for hot compress, since the hot towel cannot preserve heat, the temperature of the towel drops very quickly and finally have no hot compress effect. When a hot bag is used, the hot bag has a higher temperature just after hot water is filled in it, and the high temperature may hurt people. The temperature of the hot bag still drops after it is used for a long period.

Therefore, it is reliable to use an ice/hot bag or an ice/hot pack which can preserve heat and keep the temperature at a desired value for a long period for ice/hot compress. For the patient of diabetes who are not sensible for temperature, such ice/hot compress products are better choice for them to avoid possible hurt or failure of treatment.

Although such ice/hot compress products can keep the temperature for a longer period, its temperature is possible to change after a long-time usage and thus causes hurt for patient or failure of treatment. A thermochromic device is disposed on an outer surface of the ice/hot compress products to prevent excessively high or low temperature. However, a complicated process and higher cost are needed for manufacturing such a thermochromic device.

### BRIEF SUMMARY OF THE INVENTION

An object of the invention is to provides a manufacturing method for an ice/hot compress product, wherein the process of manufacturing the ice/hot compress product with thermochromic function is simplified.

To achieve the object of the invention, the invention provides a manufacturing method for an ice/hot compress product. The manufacturing method in accordance with an exemplary embodiment of the invention includes the following steps: providing a polyester fiber cloth as an outer layer; adding a thermochromic material into the outer layer when the outer layer is dyed; and attaching an inner layer to an inner side of the outer layer to which the thermochromic material has been added to form a composite-layer material; manufacturing a container through the composite-layer material.

In another exemplary embodiment, the inner layer is a thermoplastic polyurethane elastomer layer.

In yet another exemplary embodiment, the inner layer is a polyvinyl chloride layer.

In another exemplary embodiment, the thermochromic material is a mono thermochromic material.

In yet another exemplary embodiment, the thermochromic material is a two-stage-indication thermochromic material.

In another exemplary embodiment, the thermochromic material is a multi-stage-indication thermochromic material.

A detailed description is given in the following embodiments with reference to the accompanying drawings.

### BRIEF DESCRIPTION OF THE DRAWINGS

The invention can be more fully understood by reading the subsequent detailed description and examples with references made to the accompanying drawings, wherein:
Fig. 1 is a schematic view of an embodiment of a manufacturing method of an ice/hot compress product of the invention;
Fig. 2 is a schematic view of an embodiment of an ice/hot compress container of the invention;
Fig. 2A is an enlarged view of Fig. 2;
Fig. 3 is a schematic view of the ice/hot compress container of Fig. 2, wherein the outer surface of the ice/hot compress container indicates a higher temperature; and
Fig. 4 is a schematic view of the ice/hot compress container of Fig. 2, wherein the outer surface of the ice/hot compress container indicates a lower temperature.

### DETAILED DESCRIPTION OF THE INVENTION

The following description is of the best-contemplated mode of carrying out the invention. This description is made for the purpose of illustrating the general principles of the invention and should not be taken in a limiting sense. The scope of the invention is best determined by reference to the appended claims.

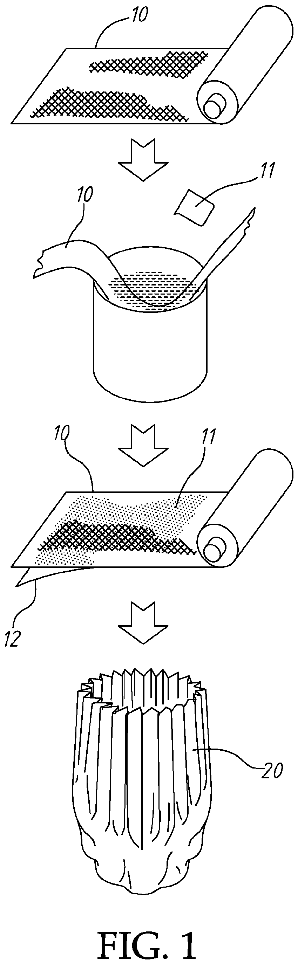

Referring to Fig. 1, a manufacturing method of an ice/hot compress product of the invention includes the following steps:
providing a polyester fiber cloth as an outer layer 10;
adding a thermochromic material 11 into the outer layer 10 when the outer layer 10 is dyed; and
attaching an inner layer12 to an inner sided of the outer layer 10 to which the thermochromic material 11 has been added to form a composite-layer material; manufacturing a container 20 through the composite-layer material.

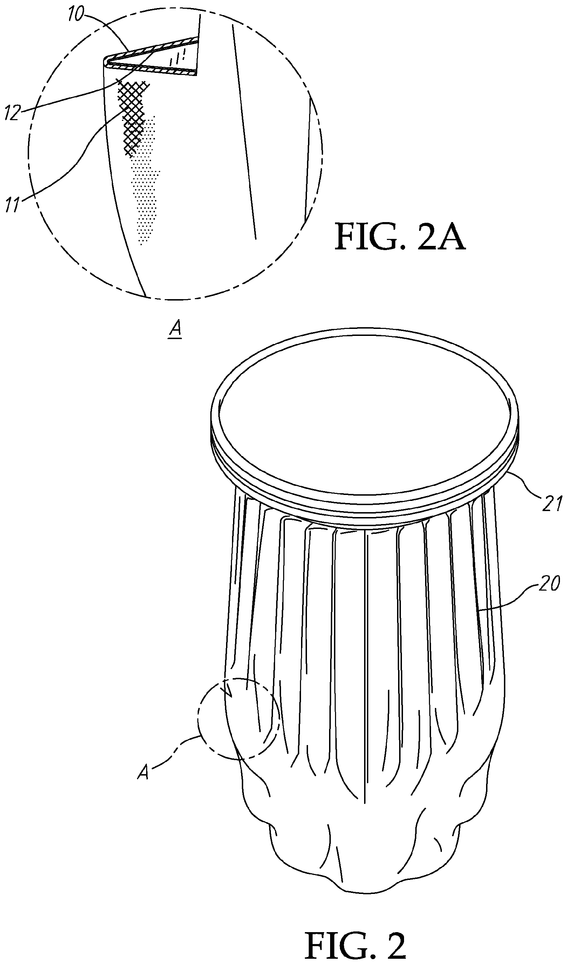

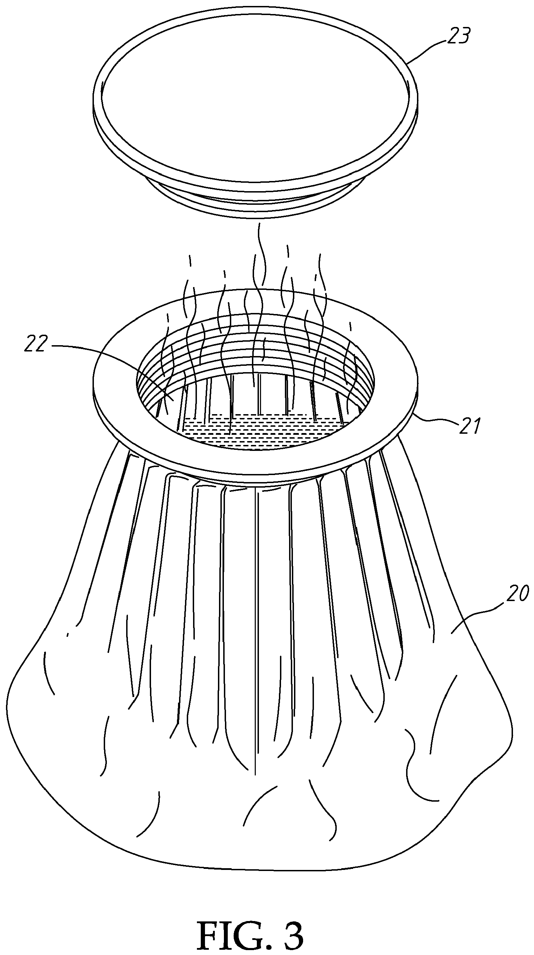

Referring to Figs. 2 and 2A, a seat 21 is mounted to a top of the container 20 to form an entrance 22 (see Fig. 3) for filler. A cover 23 is fitted to the seat 21 to close or open the entrance 22 so as to seal the container 20 or open it.

The inner layer 12 can be made of various material according to the container 20 serving as an ice/hot bag or an ice/hot pack, such as thermoplastic urethane (TPU) or polyvinyl chloride (PVC). The material can prevent leakage of filler and facilitate the combination of the outer layer 10 and the inner layer 12.

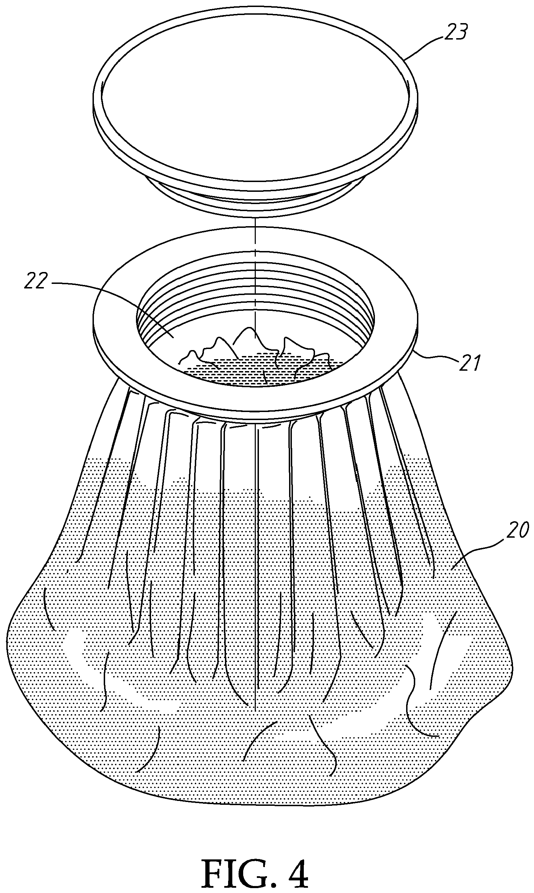

In an embodiment, the thermochromic material 11 can be a mono thermochromic material which shows a color at a low temperature (Fig. 4), but shows no color at a high temperature (Fig. 3). For example, when the temperature of the filler in the container 20 drops to 25°C, the thermochromic material 11 begins to show a color, and the color saturation reaches 100% when the temperature drops to 22°C. In another embodiment, the thermochromic material 11 can also be a two-stage-indication thermochromic material which shows a color (for example green color) at a low temperature and shows the other color (for example red color) at a high temperature. In another embodiment, the thermochromic material 11 can also be a multi-stage-indication thermochromic material which shows a color (for example purple color) at a low temperature, shows another color at a medium temperature (for example light red color) and shows another color (for example red color) at a high temperature.

The invention provides an ice/hot compress product capable of indicating temperature change of the filler in the container 20. Since the thermochromic material 11 has been added to the outer layer 10 when the outer layer 10 is dyed, it simplified the manufacturing process for the thermochromic product.

While the invention has been described by way of example and in terms of preferred embodiment, it is to be understood that the invention is not limited thereto. To the contrary, it is intended to cover various modifications and similar arrangements (as would be apparent to those skilled in the art). Therefore, the scope of the appended claims should be accorded the broadest interpretation so as to encompass all such modifications and similar arrangements.

## Claims

1. A manufacturing method for an ice/hot compress product, comprising:
providing a polyester fiber cloth as an outer layer (10);
adding a thermochromic material (11) into the outer layer (10) when the outer layer (10) is dyed; and
attaching an inner layer (12) to an inner side of the outer layer (10), to which the thermochromic material (11) has been added, to form a composite-layer material;
manufacturing a container (20) through the composite-layer material.

2. The manufacturing method as claimed in claim 1, wherein the inner layer (12) is a thermoplastic polyurethane elastomer layer.

3. The manufacturing method as claimed in claim 1, wherein the inner layer (12) is a polyvinyl chloride layer.

4. The manufacturing method as claimed in claim 1, wherein the thermochromic material (11) is a mono thermochromic material (11).

5. The manufacturing method as claimed in claim 1, wherein the thermochromic material (11) is a two-stage-indication thermochromic material (11).

6. The manufacturing method as claimed in claim 1, wherein the thermochromic material (11) is a multi-stage-indication thermochromic material (11).
